(19) Europäisches Patentamt
European Patent Office
Office européen des brevets

(11) EP 2 392 570 B1

(12) # EUROPÄISCHE PATENTSCHRIFT

(45) Veröffentlichungstag und Bekanntmachung des Hinweises auf die Patenterteilung:
**01.10.2014 Patentblatt 2014/40**

(51) Int Cl.:
***C07D 319/12** (2006.01)* ***C07B 55/00** (2006.01)*

(21) Anmeldenummer: **11004941.8**

(22) Anmeldetag: **28.08.2009**

(54) **Verfahren zur Herstellung eines Gemisches von Lactid-Derivaten**

Method for manufacturing a mixture of lactide derivatives

Procédé de fabrication d'un mélange de dérivés de lactides

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **29.08.2008 DE 102008044947**

(43) Veröffentlichungstag der Anmeldung:
**07.12.2011 Patentblatt 2011/49**

(62) Dokumentnummer(n) der früheren Anmeldung(en) nach Art. 76 EPÜ:
**09778182.7 / 2 321 294**

(73) Patentinhaber:
- **Uhde Inventa-Fischer GmbH
13509 Berlin (DE)**
- **ThyssenKrupp Uhde GmbH
44141 Dortmund (DE)**

(72) Erfinder:
- **Hagen, Rainer
13465 Berlin (DE)**
- **Verweij, Adam Bastiaan
9831 RN Aduard (NL)**
- **Mühlbauer, Udo
10119 Berlin (DE)**
- **Schulze, Joachim
59494 Soest (DE)**
- **Tietz, Wolfgang
06408 Biendorf (DE)**
- **Göhler, Klaus-Dieter
verstorben (DE)**

(74) Vertreter: **Pfenning, Meinig & Partner GbR
Patent- und Rechtsanwälte
Theresienhöhe 11a
80339 München (DE)**

(56) Entgegenhaltungen:
**EP-A- 0 657 447 EP-A- 1 577 346
US-A- 5 463 086**

- **von Vogel (Hrsg.): "Chemiker-Kalender, 2. Auflage", 1974, Springer-Verlag, Heidelberg, XP002661337, Seiten 326-327, * L-(+)-Milchsäure, D-(-)-Milchsäure. Zitiert als allgemeines Fachwissen. ***
- **ANDERSON ET AL.: "Melt Chain Dimensions of Polylactide", MACROMOLECULES, Bd. 37, Nr. 5, 1. März 2004 (2004-03-01), Seiten 1857-1862, XP55009574, ISSN: 0024-9297, DOI: 10.1021/ma0357523**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 392 570 B1

## Beschreibung

Die vorliegende Erfindung betrifft ein Verfahren zur Herstellung eines Racemates von Dilactid. Dabei wird von L-Milchsäure ausgegangen. Es findet eine Racemisierung des chiralen Kohlenstoffatoms des Eduktes statt.

Polymilchsäure ist ein vielversprechendes Biopolymer. Ein Problem für seinen Durchbruch als Massenware-Polymer ist seine geringe Wärme-Formbeständigkeit. Für den Fall, dass es möglich wäre, bessere thermische Eigenschaften zu erzielen, würden die möglichen Anwendungen stark ansteigen.

Um PLLA mit optimalen thermischen Eigenschaften herstellen zu können, wird (optisch) sehr reines L-Lactid (L-LA) benötigt. Die am gängigsten verwendete Methode zur Herstellung von L-Lactid umfasst eine zweistufige Polykondensation von Milchsäure zu einem Oligomer, an die sich eine Depolymerisation anschließt. Wegen den vorherrschenden hohen Temperaturen, die für einen schnellen Reaktionsablauf benötigt werden, sowie kationischen Verunreinigungen der Milchsäure oder der Reaktionsgefäße (z.B. durch Korrosion) besteht das Problem der Racemisierung, wodurch meso-Lactid als Nebenprodukt entsteht. Dieses Produkt muss vom Hauptprodukt abgetrennt werden, da meso-Lactid (M-LA) einen negativen Einfluss auf die Eigenschaften des bei der Polymerisation von L-Lactid entstehenden Polymers besitzt. Dabei kommt es zu einer merklichen Abnahme des Schmelzpunktes sowie der Glasübergangstemperatur, während sich ebenso die mechanischen Eigenschaften verändern.

**Tabelle 1**

| | PLLA | PRLA | PMLA (a/s) | sc-PLA | sbc-PLA |
|---|---|---|---|---|---|
| Tg °C | 55-60 | 50-55 | 40-45/34 | 80-90 | 50-55 |
| Tm °C | 140-170 | - | -/153 | 210-230 | 185-195 |

Tg: Glasübergangstemperatur
Tm: Schmelzpunkt
PLLA: L-Polymilchsäure
PRLA: racemische Polymilchsäure
PMLA: meso-Polymilchsäure
a: amorph
s: syndiotaktisch
sc: Stereo-Komplex
sbc: Stereo-Block-Copolymer

Meso-Lactid ist wie L-Lactid ein zyklischer Diester mit zwei optisch aktiven Kohlenstoffatomen im Ring. Es besitzt ein optisches R- und ein S-Zentrum und ist daher optisch inaktiv. Die Polymerisation von Meso-Lactid führt zu einem amorphen Polymer; unter Verwendung eines stereo-selektiven Katalysators ist ein syndiotaktisches Polymer (Tina M. Quitt und Geoffrey W. Coates, J. Am. Chem. Soc. 1999, 121, 4072-4073) herstellbar, dessen thermische Eigenschaften jedoch schlechter als die von PLLA sind.

Stereo-Komplexe von Polymilchsäure (PLA) können das Problem der geringen thermischen Stabilität lösen, jedoch ist zur Herstellung von Stereo-Komplexen das optische Gegenstück von L-Polymilchsäure (PLLA) nötig. D-Polymilchsäure (PDLA) ist nur in geringen Mengen verfügbar und sehr teuer.

Rac-Lactid wird bisher aus gleichen Mengen D,D- und L,L-Lactid durch Verschmelzen gewonnen. Da D,D-Lactid wegen des hohen Aufwands für die Herstellung der D-Milchsäure relativ teuer ist, war die Verwertung als Monomer für die Polymilchsäureherstellung bisher mehr von theoretischem Interesse. Dabei sind die Eigenschaften von D,D-L,L-stereo-Polymeren, hochinteressant, da sie deutlich bessere Thermostabilitäten aufweisen und damit einen der Nachteile von Polymilchsäure beseitigen könnten.

Dilactide, die aus den Enantiomeren der Milchsäure zusammengesetzt sind, sind vorbekannt. Die WO 1984/04311 A1 beschreibt ein Verfahren zur Herstellung eines Polymers aus Caprolacton und Lactid, welches zur Herstellung von Gebrauchsgegenständen in der Medizin und Pflegetechnik eingesetzt wird. Das Dilactid ist kommerziell erhältlich und ist zum überwiegenden Teil aus den beiden Enantiomeren der Milchsäure, L- und D-Milchsäure zusammengesetzt. Dieses Gemisch ist häufig mit Dilactid vergesellschaftet, welches aus gleichen Enantiomeren der Milchsäure, nämlich aus D-Milchsäure oder L-Milchsäure, besteht. Ein Hinweis zur Herstellung dieser aus gleichen Enantiomeren zusammengesetzten Dilactide wird nicht gegeben.

Die Polymerisation einer Mischung aus meso-Lactid und L-Lactid führt zu einem Copolymer, dessen thermische Eigenschaften denen von PLLA unterlegen sind. Meso-Lactid kann auch bei der Herstellung von racemischer Milchsäure (D/L-LA) durch Hydrolyse mit Wasser verwendet werden. Jedoch sind diese Anwendungen aus kommerzieller Sicht von nur untergeordnetem Interesse, so dass eine Erhöhung des ökonomischen Wertes angestrebt wird.

Die EP 0 657 447 betrifft eine Methode zur Herstellung von racemischem D/L-Dilactid, bei dem racemische Milchsäure mit SnO als Katalysator zu einer rohen Dilactid-Mischung aus D/L-Dilactid und Meso-Dilactid umgesetzt wird. Nachfolgend wird das rohe Dilactid zur Abreicherung des Meso-Dilactids zuerst aus Wasser kristallisiert und dann aus Aceton umkristallisiert.

K. S. Anderson et al. beschreiben in "Macromolecules, 2004, 37, 18 57-1862 die Racemisierung von deuterierter L-Milchsäure bei der Umsetzung von Sn(II)-2-ethylhexanoat zu deuteriertem L,L-Dilactid, während bei derselben Umsetzung nicht-deuterierter L-Milchsäure reines L,L-Dilactid erhalten wird.

Ausgehend hiervon war es somit Aufgabe der vorliegenden Erfindung, ein Verfahren bereitzustellen, das die Herstellung eines Racemates aus D,D-Dilactid und L,L-Dilactid aus L-Milchsäure ermöglicht.

Diese Aufgabe wird durch die Merkmale des Patentanspruches 1 gelöst. Bevorzugte Ausführungsformen werden in den abhängigen Patentansprüchen genannt.
Es werden drei Varianten zur Herstellung eines Gemisches der unten dargestellten Verbindungen der allgemeinen Formeln Ia, Ib und Ic vorgestellt.

Gemäß einer ersten Variante wird ein Verfahren zur Herstellung eines Gemisches aus den Verbindungen der Formeln Ia, Ib und/oder Ic bereitgestellt

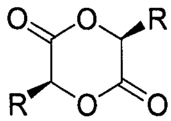
Ia
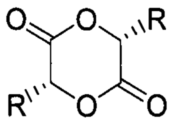
Ib
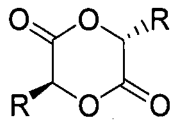
Ic

bei dem eine $\alpha$-Hydroxycarbonsäure der Formel IIa und/oder IIb

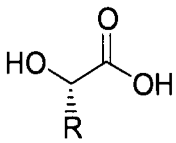
IIa
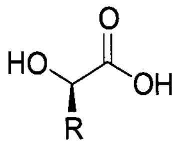
IIb

(wobei jeweils in den Verbindungen der Formeln I und II R für einen linearen oder verzweigten aliphatischen Alkylrest mit 1 bis 6 C-Atomen steht), mit einem Katalysator oder einem Gemisch aus mindestens zwei Katalysatoren umgesetzt wird.

Gemäß dieser ersten Variante zur Herstellung des Gemisches der Verbindungen Ia, Ib und/oder Ic wird somit von einer $\alpha$-Hydroxycarbonsäure der Formeln IIa und/oder IIb ausgegangen, die in Gegenwart von mehreren Katalysatoren umgesetzt wird. Dabei können sowohl jeweils die Verbindungen IIa und IIb als im Wesentlichen oder vollständig enantiomerenreine Verbindungen eingesetzt werden, es kann jedoch auch von einem Gemisch der beiden enantiomerenreinen Verbindungen in einem beliebigen stöchiometrischen Verhältnis ausgegangen werden. Unter "im Wesentlichen enantiomerenrein" wird ein Gemisch der Verbindungen IIa und IIb mit einem Enantiomeren-Überschuss (ee) von mehr als 99 % ee verstanden.

Gemäß einer zweiten Variante des Verfahrens zur Herstellung des oben genannten Gemisches der Verbindungen Ia, Ib und/oder Ic wird eine im Wesentlichen oder vollständig stereoisomerenreine Verbindung der Formel Ia, Ib oder Ic oder auch Gemische von Ia, Ib und Ic mit einem Katalysator oder einem Gemisch aus mindestens zwei Katalysatoren umgesetzt. Unter "im Wesentlichen stereoisomerenrein" wird dabei das Gemisch der Verbindungen Ia, Ib und/oder Ic verstanden, bei dem eine der genannten Verbindungen in einem Überschuss bezüglich der Summe der beiden anderen Verbindungen von mindestens 99 % vorliegt. Bei dieser Variante wird somit von einer einzelnen Verbindung der Formeln Ia, Ib oder Ic ausgegangen, wobei im Verlauf des Verfahrens eine Umwandlung der Stereozentren der eingesetzten zyklischen Diester stattfindet. Geht man von einem Gemisch der Verbindungen Ia, Ib und/oder Ic aus, erhält man wiederum ein Gemisch dieser Verbindungen, jedoch mit veränderter Zusammensetzung.

Eine dritte Variante sieht die Herstellung des Gemisches der Verbindungen Ia, Ib und/oder Ic vor, bei dem eine oligomere oder polymere Hydroxycarbonsäure der allgemeinen Formel III

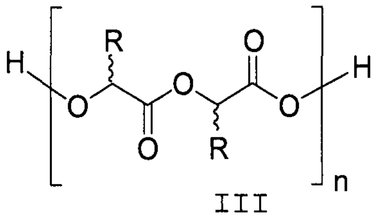

III

(wobei in der Formel III n = 1 bis 50 bedeutet), mit einem Katalysator oder einem Gemisch aus mindestens zwei Katalysatoren umgesetzt wird.

Diese dritte Variante sieht somit eine Depolymerisation von oligomerer oder polymerer Hydroxycarbonsäure der Formel III vor. Gemäß dem Verfahren der Variante III können für die Hydroxycarbonsäure der Formel III alle möglichen Stereoisomeren eingesetzt werden. Dies ist in der Formel III durch die geschlängelte Bindung des Restes R angedeutet. Unerheblich dabei ist die absolute Konfiguration an jeweiligen Stereozentrum (R oder S).

Grundsätzlich können für alle der drei oben genannten Varianten die Katalysatoren ausgewählt sein aus der Gruppe bestehend aus

a) Metallverbindungen der Gruppen 1 bis 14 des Periodensystems, bevorzugt Metallsalze und/oder metallorganische Verbindungen, Alkoxide, Oxide, Salze organischer Säuren, weiter bevorzugt Metallsalze und/oder metallorganische Verbindungen von Na, K, Mg, Ca, Fe, Ti, Zn, Sn, Sb, besonders bevorzugt deren Oxide, Hydroxide, Carbonate, Benzoate, Lactate, Octoate, insbesondere MgO, CaO, $K_2CO_3$, Natriumlactat, Kaliumbenzoat, Zinnoctoat $SnOc_2$, Dibutylzinnoxid $Bu_2SnO$, $BuSnOc_3$, SnO und/oder

b) stickstoffhaltigen oder phosphorhaltigen organischen Verbindungen, bevorzugt primären, sekundären und/oder tertiären Aminen und/oder aliphatischen, aromatischen N-heterocyclischen Verbindungen mit 5-7 Ringatomen oder Phosphinen, weiter bevorzugt primären, sekundären und/oder tertiären Aminen mit 1 bis 20 C-Atomen, besonders bevorzugt Triethylamin, Ethyldiisopropylamin, Dibutylamin, Tributylamin, Trioctylamin, Dicyclohexylamin, 4-(N,N-Dimethyl)-aminopyridin, 2,2,6,6-Tetramethylpiperidin, 1,2,2,6,6-Pentamethylpiperiden und/oder Tributylphosphin.

Bevorzugt werden bei den drei Varianten drei Arten von Katalysatoren unterschieden:

1. Polymerisationskatalysatoren
2. Racemisierungskatalysatoren
3. Stereoselektive Katalysatoren.

1. Als Polymerisationskatalysatoren für PLA sind eine Vielzahl von Verbindungen bekannt. Es sind häufig Metall- oder Organo-Metall-Salze wie Alkoxide, Oxide, Salze organischer Säuren etc. Meistens wird Zinnoctoat ($SnOc_2$) eingesetzt. Weiterhin werden auch andere Zinn-Verbindungen wie z.B. $BuSnOc_3$, $Bu_2SnO$, SnO oder auch Zinn eingesetzt. Auch der Einsatz von Verbindungen des Ti, Fe, Zn, Sb etc. ist möglich.

2. Racemisierungskatalysatoren, die zur Racemisierung von Lactiden eingesetzt werden, sollten schwach alkalische Verbindungen sein und keine Ringöffnungspolymerisation (ROP) bewirken. Hier wurden drei Verbindungs-Klassen gefunden:

a) Gruppe 1a und 2a Metalloxide, Carbonate, Hydroxide oder Salze von organischen Säuren wie z.B. Natrium-Lactat, Kalium-Benzoat, $K_2CO_3$, MgO, CaO etc.
b) Amine, primäre, sekundäre oder tertiäre Amine mit einem Siede- oder Schmelzpunkt, der hoch genug ist, dass die Verbindung in der Reaktion verbleibt. Bevorzugt sind hier sekundäre oder tertiäre Amine wie z.B. Triethylamin (TEA), Tributylamin (TBA), Trioctylamin (TOA), Dibutylamin (DBA), Di-Cyclohexylamin (DCHA), Dimethylamino-Pyridin (DMAP) etc.
c) Primäre, sekundäre oder tertiäre Phosphine.

Für alle hier genannten flüchtigen Katalysatoren gilt, dass der Siedepunkt hoch genug sein muss, dass die Verbindung in der Reaktion verbleibt.

Bei der Auswahl des Racemisierungs-Katalysators ist zu beachten, dass der Katalysator nur eine Racemisierung und keine Ringöffnung des Lactids katalysiert. Diese beiden Konkurrenzreaktionen hängen von der chemischen und sterischen Struktur des Katalysators ab. Eine Ringöffnung macht die Trennung einer Lactid-Mischung nach der Racemisierung schwieriger und senkt die Ausbeute. Die Reinheit der racemischen Lactid-Mischung nach der Trennung

ist für eine stereo-selektive Katalyse zur Erzeugung von sc-PLA und sbc-PLA wichtig.

Wegen einer sterische Hinderung des aktiven Zentrums sind sekundäre und tertiäre Amine und Phosphine bevorzugte Katalysatoren. Besonderes bevorzugt sind voluminöse organische Reste wie z.B. die Cyclohexylgruppe in DCHA.

Die Ringöffnung wird durch sie behindert und ihre schwache alkalische Aktivität ist für die Racemisierung ausreichend. Dennoch trifft auf alle genannten Katalysatoren zu, dass sie ihre Selektivität bei hohen Temperaturen bzw. langen Reaktionszeiten verlieren.

3. Stereoselektive Katalysatoren (Spassky et all, Macromol.Chem.Phys.(1996),197, 2672; Ovitt and Coates, J.Am.Chem.Soc.,(2002),124, 1316; Radano and Baker, J.Am.Chem.Soc.,(2000),122, 1552) sind sehr spezifische Polymerisations-Katalysatoren, die ein chirales Zentrum aufweisen. Sie katalysieren ausschließlich die Polymerisations-Reaktion- von spezifischen Isomeren. Hier werden verschiedene Typen unterschieden. Eine Klasse dieser Katalysatoren kann nur die Reaktion des Isomers (L,L/D,D-Lactid + ssc -> PLLA + D,D-Lactid) katalysieren, wohingegen ein anderer Typ mit zwei aktiven Zentren zwei Isomere gleichzeitig polymerisieren kann (L,L/D,D-Lactid + ssc -> PLLA + PDLA = sc-PLA). Es sind auch Katalysatoren bekannt, die L- oder D-Lactid alternierend polymerisieren können (L,L/D,D-Lactid + ssc -> $(PLLA\text{-}co\text{-}PDLA)_n$ = sbc-PLA).

Weiter ist bei den oben genannten Varianten jeweils vorteilhaft, wenn der Katalysator bezüglich der jeweiligen Edukte der verschiedenen Varianten, also der $\alpha$-Hydroxycarbonsäure der Formel IIa und/oder IIb, der im wesentlichen stereoisomerenreinen oder stereoisomerenreinen Verbindung der Formel Ia, Ib oder Ic oder einem Gemisch aus zwei oder drei der Verbindungen, oder der oligomeren oder polymeren Hydroxycarbonsäure der allgemeinen Formel III in einem Gewichts-Verhältnis zwischen 1:1 und 1:10.000, bevorzugt zwischen 1:10 und 1:5.000, besonders bevorzugt zwischen 1:100 und 1:1.000 eingesetzt wird.

Überraschenderweise konnte festgestellt werden, dass bevorzugt das beim Verfahren erhaltene molare Verhältnis der Verbindungen der Formel Ia und Ib zwischen 1:2 und 2:1 bevorzugt zwischen 1:1,2 und 1,2:1, besonders bevorzugt im Wesentlichen 1:1 beträgt.

Weiterhin wurde überraschenderweise gefunden, dass vorzugsweise das beim Verfahren erhaltene molare Verhältnis der Summe der Verbindungen der Formel Ia und Ib zur Verbindung der Formel Ic

$$(Ia+Ib)/Ic$$

zwischen 10:1 und 1:1, bevorzugt zwischen 10:1 und 2:1 beträgt.

In einer weiteren vorteilhaften Ausführungsform wird die Umsetzung bei Temperaturen zwischen 80 und 300 °C, bevorzugt zwischen 100 und 200 °C, besonders bevorzugt zwischen 120 und 160 °C durchgeführt.

Vorzugsweise wird die Umsetzung über einen Zeitraum zwischen 1 min und 48 Std., bevorzugt zwischen 0,5 und 4 Std. durchgeführt.

Besonders bevorzugt ist, wenn sich im Anschluss an die oder gleichzeitig während der Umsetzung mindestens ein Reinigungsschritt des durch die Umsetzung erhaltenen Gemisches der Verbindungen der Formeln Ia, Ib und/oder Ic anschließt bzw. durchgeführt wird, wobei das Verhältnis der Summe der Verbindungen der Formel Ia und Ib zur Verbindung der Formel Ic

$$(Ia+Ib)/Ic$$

auf mindestens 10:1, bevorzugt auf mindestens 100:1, weiter bevorzugt auf mindestens 1.000:1 erhöht wird, insbesondere die Verbindung der Formel Ic im Wesentlichen vollständig oder vollständig entfernt wird. Unter "im Wesentlichen vollständige Entfernung" wird dabei eine Reduktion des Gehaltes der Verbindung Ic auf Konzentrationen im ‰-Bereich verstanden.

Gemäß dieser bevorzugten Ausführungsform des Verfahrens wird somit die Herstellung eines Gemisches, das lediglich aus den Verbindungen der Formeln Ia und Ib besteht, ermöglicht. Besonders bevorzugt ist dieses Gemisch ein Racemat, also ein äquimolares Gemisch der Verbindungen Ia und Ib, das als racemisches Lactid bezeichnet wird.

Weiter ist der zuvor genannte Reinigungsschritt dabei ausgewählt aus der Gruppe bestehend aus Filtration, Waschen, Destillation, Kristallisation und/oder Umkristallisation des Gemisches der Verbindungen der Formel Ia, Ib und/oder Ic, sowie Kombinationen aus den genannten Reinigungsschritten. Kombinationen können dabei eine nacheinander abfolgende oder gleichzeitige Durchführung der zuvor genannten Reinigungsmethoden sein. Beispielsweise kommen hierzu eine Filtration oder Waschen des bei der Umsetzung erhaltenen Gemisches, gefolgt von einer Destillation oder einer Kristallisation in Frage; ebenso ist jedoch beispielsweise eine Destillation gefolgt von einer Kristallisation möglich.

Die Kristallisation und/oder Umkristallisation kann aus der Schmelze oder aus Lösungsmitteln durchgeführt werden, wobei das Lösungsmittel bevorzugt ausgewählt ist aus der Gruppe von Alkoholen, Estern, Ketonen, Kohlenwasserstoffen usw., z.B. Aceton, iso-Propanol, Ethylacetat, Toluol und/oder Kombinationen hieraus. Bevorzugt wird das erhaltene Rohprodukt aus Ia, Ib und Ic durch Umkristallisation aus der Schmelze gereinigt, wobei Ia und Ib als Reinprodukt auskristallisieren.

**Tabelle 2:** Schmelztemperaturen der Lactide

| | L,L-Lact. | D,D-Lact. | M-Lact. | L, L/D, D-Lact. |
|---|---|---|---|---|
| Tm °C | 97 | 97 | 54 | 129 |

Nach der Abtrennung der bei der Kristallisation zurückbleibenden Schmelze, die Verbindung Ic gegebenenfalls im Gemisch mit Ia und/oder Ib enthält, kann diese in die Reaktionsstufe zurückgeführt werden. Auf diese Weise lässt sich z.B. eine vollständige Umwandlung von Ic in ein äquimolares Gemisch aus Ia und Ib überführen.

Bevorzugt kann gemäß Variante 1) eine im Wesentlichen enantiomerenreine oder enantiomerenreine Verbindung der Formel IIa oder IIb eingesetzt werden.

Das erfindungsgemäße Verfahren betrifft die Herstellung eines äquimolaren Gemisches aus den beiden Enantiomeren des Dilactides der Milchsäure, D,D-Dilactid und L,L-Dilactid, bei dem

a) L-Milchsäure mit Trioctylamin zu Trioctylammoniumlactat umgesetzt wird,
b) das Trioctylammoniumlactat in Gegenwart eines Katalysators destilliert wird, wobei eine Fraktion erhalten wird, die im wesentlichen aus den beiden Enantiomeren des Dilactides der Milchsäure, D,D-Dilactid und L,L-Dilactid, zusammengesetzt ist und noch D,L-Lactid enthalten kann
c) wobei die zuvorgenannte Fraktion mit Aceton versetzt und damit umkristallisiert wird, und farblose Kristalle mit einem Schmelzpunkt von 112 bis 119 °C erhalten werden, die äquimolar aus D,D-Dilactid und L,L-Dilactid zusammengesetzt sind.

Erfindungsgemäß wird somit die Herstellung eines Racemates aus L,L-Dilactid und D,D-Dilactid ermöglicht. Die Umsetzung (Reaktion mit dem Katalysator) und der erste Reinigungsschritt (Destillation) laufen dabei simultan ab.

Erfindungsgemäße Verwendungsmöglichkeiten des nach dem erfindungsgemäßen Verfahren hergestellten Gemisches sind beispielsweise die sich (z.B. unmittelbar an das erfindungsgemäße Verfahren) anschließende Herstellung von amorphen Polylactiden und insbesondere die Herstellung von stereokomplexer Polymilchsäure und/oder Stereo-Block-Copolymeren der Milchsäure mit stereo-selektiven Katalysatoren.

Die Erfindung betrifft insbesondere ein Verfahren zur Herstellung eines äquimolaren Gemisches aus D,D-Dilactid und L,L-Dilactid, wobei man zur Herstellung dieser Substanzen von L-Milchsäure ausgeht, die mit Trioctylamin zu Trioctylammoniumlactat umgesetzt wird, welches einer destillativen Spaltung (im Sinne einer Kondensation zweier Milchsäureeinheiten unter Racemisierung) unterworfen wird, wobei man ein Destillat erhält, das aus Aceton umkristallisiert wird und so das erfindungsgemäße Dilactid erhalten wird.

Es wurde nun gefunden, dass sich das Dilactidgemisch, welches zu gleichen Teilen aus dem D,D-Dilactid und dem L,L-Dilactid besteht, auf einfache Weise herstellen lässt, wenn das Destillat, welches aus der Thermolyse des Ammoniumlactats erhalten wird, einer Umkristallisation unterworfen wird. Das so erhaltene Dilactidgemisch besitzt wesentliche Vorteile bei der Herstellung von Polymeren aus Milchsäure, die verbesserte physikalische Eigenschaften aufweisen.

Zur Herstellung dieses Dilactides geht man von L-Milchsäure aus, die mit Trioctylamin zu Trioctylammoniumlactat umgesetzt wird. Bevorzugt wird als Trioctylamin das Tri-n-octylamin eingesetzt. Dabei bildet sich das Trioctylammoniumlactat, welches einer destillativen Spaltung (im Sinne einer Kondensation zweier Milchsäureeinheiten unter Racemisierung) unterzogen wird. Bei der destillativen Spaltung erhält man ein Gemisch, welches aus Milchsäure und Trioctylamin besteht. Eine weitere Fraktion besteht aus Dilactid, welches zunächst wasserklar und dann zunehmend gelb überdestilliert. Die destillative Spaltung (im Sinne einer Kondensation zweier Milchsäureeinheiten unter Racemisierung) findet in Gegenwart eines Katalysators statt. Die Fraktion aus der destillativen Spaltung, die überwiegend aus Dilactid besteht, wird nach dem Erkalten umkristallisiert. Als Lösungsmittel wird bevorzugt Aceton eingesetzt. Dabei erhält man farblose Kristalle, die einen Schmelzpunkt von 112 °C bis 119 °C aufweisen.

Die erhaltenen Kristalle wurden einer Analyse durch Gaschromatographie unterzogen, wobei eine chirale Trennsäule eingesetzt wurde. Bei der Analyse wurden zwei flächengleiche Signalspitzen beobachtet, die dem D,D-Dilactid und dem L,L-Dilactid zugeordnet werden konnten. Die stereochemische Konfiguration der Dilactide konnte durch eine enzymatische Hydrolyse der Dilactide bestätigt werden, bei der ein massengleich zusammengesetztes Gemisch von Milchsäure jeweils einer stereochemischen Konfiguration erhalten wurde. Im ungereinigten Destillat beobachtet man

bei der Hydrolyse bei der gaschromatographischen Analyse ein weiteres, deutlich schwächeres Signal, welches dem Mesolactid (Dimer aus D- und L-Milchsäure) zugeordnet wurde.

Beansprucht wird somit insbesondere ein Verfahren zur Herstellung eines äquimolaren Gemisches aus den beiden Enantiomeren des Dilactides der Milchsäure, wobei

- man zunächst aus L-Milchsäure und Trioctylamin Trioctylammoniumlactat herstellt, und
- das so erhaltene Trioctylammoniumlactat einer destillativen Spaltung unterworfen wird, (wobei ein Gemisch aus Trioctylamin und Milchsäure überdestilliert), und
- die destillative Spaltung des Trioctylammoniumlactats in Gegenwart eines Katalysators durchgeführt wird, und
- man eine weitere Fraktion erhält, die zu einem überwiegenden Anteil aus dem Dilactid der Milchsäure zusammengesetzt ist, und die noch D,L-Dilactid enthalten kann, und
- diese Fraktion mit Aceton versetzt und damit umkristallisiert wird, so dass man farblose Kristalle mit einem Schmelzpunkt von 112 bis 119 °C erhält, die äquimolar aus D,D-Dilactid und L,L-Dilactid zusammengesetzt sind.

Die Bildung der enantiomerengleichen Dilactide aus enantiomerenreiner Milchsäure als Ausgangsmaterial kann dadurch erklärt werden, dass der Gehalt an Trioctylamin während der Destillation eine Racemisierung der Milchsäure bewirkt, die dann ein racemisches Trioctylammoniumlactat bildet, welches nach der destillativen Spaltung bevorzugt zum enantiomerengleichen Dilactid kristallisiert. Das Ausgangsmaterial besaß eine Enantiomerenreinheit von ca. 1 Massenprozent an D-Milchsäure.

Beansprucht wird auch ein Verfahren, bei dem sich ein Gemisch bildet, welches zu jeweils gleichen Teilen aus 40 bis 50 Massenprozent D,D-Dilactid und L,L-Dilactid zusammengesetzt ist und welches als restlichen Bestandteil beispielhaft Mesolactid (D,L-Dilactid) enthält. Je nach Herstellung kann der restliche Mengenanteil auch aus Oligolactiden oder weiteren Produkten der destillativen Spaltung bestehen.

Bevorzugt wird die destillative Spaltung des Trioctylammoniumlactats in Gegenwart eines Katalysators durchgeführt. Besonders geeignet sind für hierfür Organozinnverbindungen. Beispielhaft wird als Katalysator Dibutylzinnoxid in einer Dosierung von 0,1 bis 1 Massenprozent, bezogen auf das Gemisch im Destillationssumpf bei der destillativen Spaltung, eingesetzt. Je nach gewünschter Reinheit und Ausbeute des Dilactides wird die destillative Spaltung in Gegenwart einer Destillationskolonne durchgeführt. Bei Verwendung einer Destillationskolonne ist es vorteilhaft, die Destillation im Vakuum (z.B. 20 mbar) durchzuführen.

Bei der destillativen Spaltung erhält man einen Vorlauf, der aus Milchsäure und Trioctylamin unbekannter Zusammensetzung besteht. Dieser entspricht einem Mengenanteil von 30 bis 35 Massenprozent der Ausgangsmenge an Trioctylammoniumlactat. Dieser Anteil ist abhängig von der Verdampfertemperatur. Im Destillat wurden Amingehalte von 15 Massenprozent (140°C) bis 25 Massenprozent (165 °C) gemessen. Offensichtlich sind neben der Milchsäure und Amin auch noch gewisse Mengenanteile an Oligolactiden im Destillat enthalten. Der restliche Mengenanteil destilliert als Dilactid über. Im Destillationssumpf verbleiben etwa 2 bis 3 Massenprozent einer dunkelbraunen Flüssigkeit.

Zudem wird auch der Stoff, der durch das erfindungsgemäße Verfahren hergestellt wird, beschrieben. Es handelt sich dabei um das Dilactid der Milchsäure, welches dadurch gekennzeichnet ist, dass es sich um ein Gemisch handelt, welches zu jeweils gleichen Teilen aus 50 Massenprozent D,D-Dilactid und L,L-Dilactid zusammengesetzt ist und welches mit dem erfindungsgemäßen Verfahren hergestellt wird. Je nach Herstellungsprozess kann das Gemisch auch Verunreinigungen enthalten. Das durch das erfindungsgemäße Verfahren hergestellte Dilactid der Milchsäure zeichnet sich dadurch aus, dass es sich um ein Gemisch aus D,D-Dilactid und L,L-Dilactid und weiteren Bestandteilen handelt.

Die Ausführung des erfindungsgemäßen Verfahrens wird durch ein allgemeines Herstellungsschema und experimentelle Beispiele erläutert, wobei diese Beispiele nur typische Ausführungsformen darstellen.

## Allgemeines Herstellungsschema

Während der Herstellung von Lactid erfolgt eine Racemisierung und zusätzlich zum gewünschten R-LA wird das M-LA gebildet. Die Racemisierung findet am Lactid-Molekül statt. Das Proton am asymmetrischen Kohlenstoffatom ist empfindlich gegenüber schwach alkalischen Verbindungen und wird in einer Gleichgewichtsreaktion entfernt. Durch das Ersetzen des Protons kann das stereogene Zentrum geändert werden und eine andere sterische Konfiguration gebildet werden.

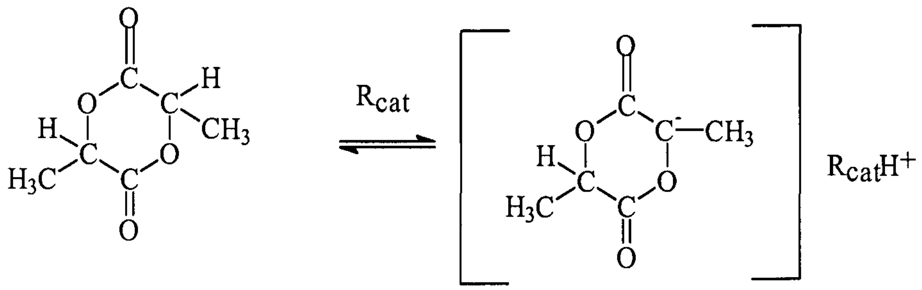


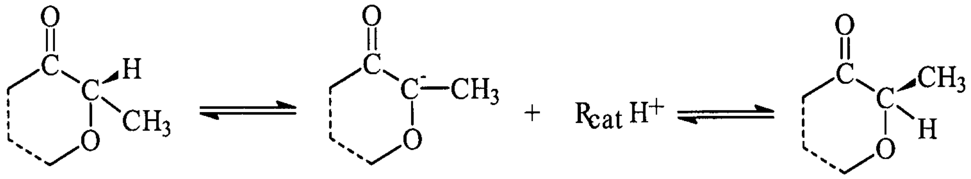

Für eine direkte Herstellung von rac-Lactid kann von L-, D- oder D,L-Milchsäure ausgegangen werden. Gemäß der Erfindung wird von L-Milchsäure ausgegangen. Nach der Entwässerung der Milchsäure wird als Katalysator eine Zinn-Verbindung zugegeben, wie sie bereits beschrieben wurden, sowie eine schwach alkalische Verbindung und dann die Destillation gestartet. Es wurde sowohl ein rac-Lactid als auch eine weitere Verbindung gefunden, die als meso-Lactid identifiziert wurde.

**Schema A: LAC -> PLA -> LA**

LAC = Milchsäure
PLA = Poly-Milchsäure
LA = Lactid

Schema A1

$$\text{LAC} \xrightarrow[R_{Kat}]{P_{kat}} \text{R-LA + M-LA}$$

LAC = L-LAC, D-LAC, D,L-LAC, LAC-Ester, Mischungen
$P_{Kat}$ = Polymerisationskatalysator
$R_{Kat}$ = Racemisierungskatalysator

Als nächster Schritt wurde eine Reaktion mit einem PLA mit niedrigem Molekulargewicht und den oben genannten Katalysatoren durchgeführt. Auch hier wurde ein identisches Reaktionsprodukt erhalten, also eine Mischung aus rac-Lactid und meso-Lactid.

Schema A2

$$\text{PLA} \xrightarrow[R_{Kat}]{P_{kat}} \text{R-LA + M-LA}$$

PLA = PLLA, PDLA, PRLA, PMLA, Mischungen, Copolymere

Weiterhin wurde eine Reaktion analog mit L-, D- und meso-Lactid durchgeführt. In jeder dieser Reaktionen wurde eine Lactid-Mischung erhalten.

Schema A3

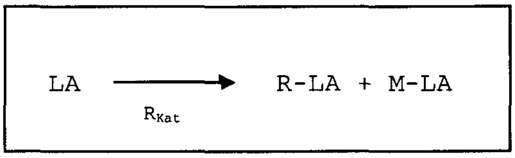


LA = L-LA, D-LA, M-LA, Mischungen aus diesen Lactiden

Diese Methode zur Herstellung von rac-Lactid, bei der alle möglichen Arten von Milchsäure bzw. deren Derivaten eingesetzt werden, ermöglicht es, ein Monomer für die Polymerisation von PLA ohne Materialverlust herzustellen, da das meso-Lactid nach der Abtrennung von rac-Lactid bei der Racemisierung wieder verwendet werden kann.

**Schema B: Verfahren**

Schema B1

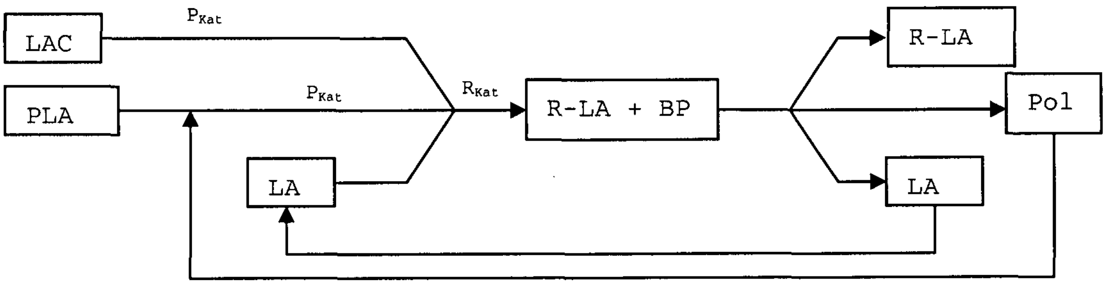


BP = Nebenprodukte
Pol = Polymer

Schema B2

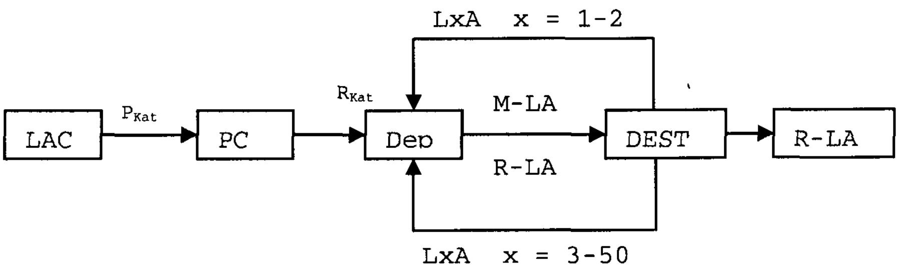


PC = PLA mit niedrigem Molekulargewicht
Dep = Depolymerisation
DEST = Destillation

Schema B3

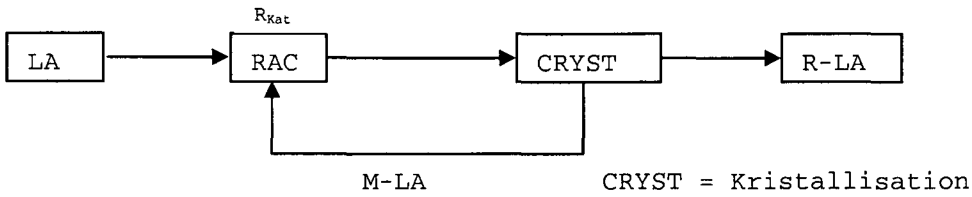


Somit ist es möglich, durch die Verfügbarkeit von stereo-selektiven Katalysatoren, sc-PLA oder sbc-PLA herzustellen, das eine höhere Hitzestabilität aufweist, ohne dass separate Produktionsmöglichkeiten für optisch reines L-Lactid und D-Lactid und deren Polymere PLLA und PDLA gegeben sind, die bisher erforderlich waren, um stereo-Komplexe herzustellen.

**Polymer-ProduktionS-Kette:**

Bisher:

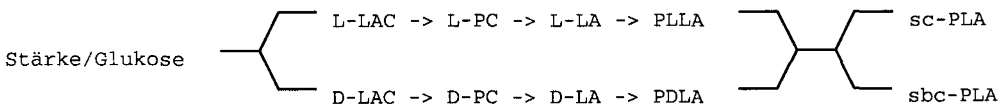


Neu:

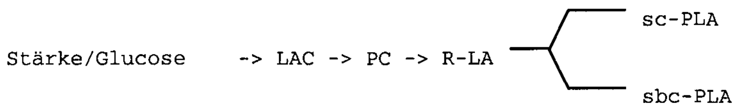


PFD Polymer-Kette

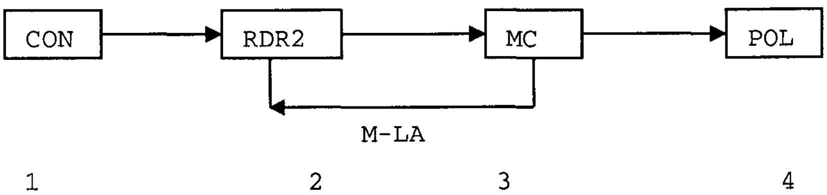


| | |
|---|---|
| CON | LAC-Konzentrator |
| RDR2 | Reaktiver Destillations- und Racemisierungs-Reaktor |
| MC | Schmelz-Kristallisierer |
| POL | Polymerisationseinheit |

Analysemethoden:

$^1$H-NMR NMR-Spektren wurden mit einem 500 MHz Varian-Inova Spektrometer bei einer Frequenz von 499,85 MHz

aufgenommen. Die Proben werden in einer ca. 5 %igen $CDCl_3$-Lösung mit Tetramethylsilan als internem Standard gemessen

HPLC Knauer system mit einer Smartline 1000 Pumpe und einem Smartline 2500 UV-Detektor. Eine Eurocel 03 Säule 5 μm 250 x 4,6 mm. Lösungsmittel Hexan: Ethanol = 90:10 (v:v) 1 ml/min. Proben-Konzentration 1-10 mg/ml.

GC Perkin-Elmer Clarus 500 mit einem FID; einer FS-CW20M-CB-0,25 Säule (Länge = 25 m, Durchmesser = 0,25 mm, Filmdicke = 0,22 μm) inj. 200 °C temp.prog. 50 - 200 °C, inj.vol. = 1,0 μl, Gas = Stickstoff.

**Beispiel 1:**

In ein Mischgefäß wird Trioctylamin und L-Milchsäure (Variante 1) mit einer optischen Reinheit von 99 % L-Milchsäure durch Erhitzen zu Trioctylammoniumlactat umgesetzt. Dieses wird in eine Destillationsvorlage gegeben, die mit einem absteigenden Liebigkühler und einem Thiele-Anschütz-Vorstoß ausgerüstet ist. In die Vorlage werden weiterhin 1 Massenprozent (bezogen auf Milchsäure) Dibutylzinnoxid als Katalysator gegeben.

Dann wird auf 250 °C erhitzt. Zunächst werden zwei Fraktionen bei 140 °C und 165 °C Destillationstemperatur erhalten, die gemäß einer gaschromatographischen Analyse aus 15 Massenprozent (140°C) und 25 Massenprozent (165 °C) Amin bestehen. Danach destilliert eine wasserklare Flüssigkeit über, die sich im Verlauf der Destillation gelb färbt. Die Flüssigkeit wird abgekühlt und in Aceton gegeben, woraus farblose Kristalle vom Schmelzpunkt 112 °C bis 119 °C kristallisieren. Deren Zusammensetzung wird durch GC-Analyse und hydrolytische Spaltung durch Enzyme als zu jeweils 50 Massenprozent aus D,D-Dilactid und L,L-Dilactid der Milchsäure bestimmt.

**Beispiel 2 (vergleich):**

Eine weitere Variante sieht insbesondere vor, im Wesentlichen enantiomerenreines Dilactid oder meso-Dilactid zu racemisieren ebenso Gemische von L-Lactid und/oder D-Lactid und/oder meso-Lactid durch Racemisierung umzusetzen (Variante 2).

Es wurde gefunden, dass die oben angegebenen Katalysatoren reines meso-Lactid oder Mischungen aus meso-Lactid und L,L-Lactid in ihrer Zusammensetzung verändern. Nach Aufreinigung und Analyse wurde festgestellt, dass racemisches Lactid erhalten wurde, folglich hatte der Katalysator meso-Lactid in racemisches Lactid überführt.

Racemisches Lactid durch Polykondensation und anschließende Depolymerisation kann aus racemischer Milchsäure hergestellt werden. Problematisch dabei ist einerseits die große Menge an meso-Lactid, die als Nebenprodukt entsteht (40-60 %) und die Verfügbarkeit sowie der Preis des Milchsäure-Racemats.

Die Racemisierung von meso-Lactid in racemisches Lactid bietet eine große Chance, um den Wert des meso-Lactids zu erhöhen, da mit verfügbaren stereo-selektiven Katalysatoren die Herstellung von sc-PLA und/oder sbc-PLA möglich ist. Diese Produkte sind aus ökonomischer Sicht sehr interessante Materialien, da sie gute thermische Eigenschaften verglichen mit PLLA aufweisen.

Von dieser Erkenntnis inspiriert testeten die Erfinder eine große Anzahl von Verbindungen auf ihre katalytische Aktivität in dieser Reaktion und fanden überraschenderweise heraus, dass mehrere Klassen von Verbindungen Wirkung zeigten. Auch für die Reaktion von L-Lactid und D-Lactid wurden die Verbindungen auf ihre Aktivität hin untersucht; auch hier konnte Racemisierung festgestellt werden.

**Beispiel 3:**

In einem Rundkolben mit Destillationsaufsatz, LiebigKühler und Thiele-Anschütz-Vorstoß wird reine L-Milchsäure 99% und Trioctylamin gemischt. Nach der Zugabe von 1 Gew.% Dibutylzinnoxid (bezogen auf die Milchsäure) wird die Mischung auf 250 °C erwärmt und zwei Fraktionen gesammelt, eine bei T < 140 °c und eine bei T = 140 - 165 °C. Mittels GC-Analyse wird ein Amin-Gehalt von 15 und 25 Gew.% bestimmt. Die dritte Fraktion ist eine klare Flüssigkeit aus der farblose Kristalle gewonnen werden können. Der Schmelzpunkt der Kristalle beträgt 112 - 119 °C und mittels GC-Analyse und einer Hydrolyse mit Enzymen wird eine 50:50-Mischung von D,D-Lactid und L,L-Lactid festgestellt (Variante 1).

**Beispiel 4 (Vergleich):**

Ein Destillationsaufbau wird mit 396,8 g L-LAC und 2,93 g KOH befüllt. Nach der Entfernung von Wasser mittels Vakuum wird 0,506 g $SnOc_2$ hinzugegeben; die Temperatur wird von 150 °C auf 240 °C erhöht und der Druck auf 10 mbar erniedrigt. Drei Fraktionen, die in einem Temperaturbereich von 100 °C bis 150 °C gesammelt werden, enthalten

die Verbindungen. Die Gesamtausbeute beträgt 46 % (L-La:D-LA:M-LA = 54:18:28)(Variante 1).

**Beispiel 5 (Vergleich):**

Ein Destillationsaufbau wird mit 253 g PLLA (Mn = 750), 0,97 g Acima TW-30 ($SnOc_2$) und 2,53 g $K_2CO_3$ befüllt. Die Temperatur wird auf 210 °C erhöht und der Druck auf 10 mbar gesenkt. Eine Fraktion wird in einem Temperaturbereich von 140 °C bis 148 °C gesammelt, wobei eine Analyse eine Zusammensetzung 33 % L-Lactid, 30 % D-Lactid und 37 % meso-Lactid bei einer Aubeute von 75 % ergibt (Variante 3).

**Beispiel 6 (Vergleich):**

In einem Headspace-Gläschen werden das Lactid und der Racemisierungskatalysator vermischt und auf 105 °C bis 155 °C erwärmt. Nach 1 bis 6 Stunden wird die Reaktion durch Kühlen des Kolbens gestoppt. Das Reaktionsprodukt wird durch $^1$H-NMR analysiert.

Die genauen Reaktionsbedingungen sowie Edukte und verwendeten Katalysatoren sind in den Tabellen 3 bis 7 angegeben.

**Tabelle 3**

| Racemisierung von meso-Lactid | | | | | | | |
|---|---|---|---|---|---|---|---|
| Exp. Nr. | Temp. °C | Zeit H | Kat. | Mol Lac./Kat. | Monomer | % m-LACT* | % L,L/D,D-LACT* |
| 1 | 140 | 4 | $K_2CO_3$ | 824 | 82 | 37 | 63 |
| 2 | 140 | 6 | $K_2CO_3$ | 825 | 77 | 27 | 73 |
| 3 | 140 | 6 | TBA | 679 | 94 | 30 | 70 |
| 4 | 140 | 6 | DCHA | 693 | 95 | 34 | 66 |
| 5 | 140 | 24 | DCHA | 690 | 87 | 24 | 76 |
| 6 | 140 | 6 | DMAP | 709 | 84 | 27 | 73 |
| 7 | 140 | 24 | DMAP | 708 | 20 | 20 | 80 |
| 8 | 140 | 6 | TBP | 718 | 94 | 47 | 53 |
| 9 | 140 | 24 | TBP | 690 | 89 | 31 | 69 |
| TBA = Tributylamin<br>DCHA = Dicyclohexylamin<br>DMAP = Dimethylaminopyridin<br>TBP = Tributylphosphin<br>* NMR-Daten; meso-Lactid: 97 % meso, 3 % L | | | | | | | |

**Tabelle 4**

| Racemisierung von meso-Lactid | | | | | | | |
|---|---|---|---|---|---|---|---|
| Exp. Nr. | Temp. °C | Zeit h | Kat. | Mol Lac./Kat. | Monomer | % m-LACT* | % L,L/D,D-LACT* |
| 1 | 125 | 1 | DCHA | 124 | § | 22 | 78 |
| 2 | 125 | 2 | DCHA | 124 | § | 19 | 81 |
| 3 | 125 | 3 | DCHA | 124 | § | 18 | 82 |
| 4 | 125 | 1 | TMPIP | 98 | § | 26 | 74 |
| 5 | 125 | 1 | PMPIP | 108 | § | 37 | 63 |
| 6 | 125 | 1 | EDiPA | 90 | § | 37 | 63 |
| 7 | 125 | 1 | TBA | 129 | § | 42 | 58 |
| 8 | 125 | 1 | TOA | 245 | § | 37 | 63 |

(fortgesetzt)

| Racemisierung von meso-Lactid | | | | | | | |
|---|---|---|---|---|---|---|---|
| Exp. Nr. | Temp. °C | Zeit h | Kat. | Mol Lac./Kat. | Monomer | % m-LACT* | % L,L/D,D-LACT* |
| 9 | 125 | 1 | $K_2CO_3$ | 96 | & | 17 | 83 |

§ = keine Nebenprodukte,
& = Nebenprodukte
TMPIP = 2,2,6,6-Tetramethylpiperidin
PMPIP = 1,2,2,6,6-Pentamethylpiperidin
EDiPA = Ethyldiisopropylamin
TBA = Tributylamin
TOA = Trioctylamin
* NMR-Daten; meso-Lactid: 90 % meso, 10 % L

**Tabelle 5**

| Racemisierung von meso-Lactid | | | | | | | |
|---|---|---|---|---|---|---|---|
| Exp. Nr. | Temp. °C | Zeit h | Kat. | Mol Lac./Kat. | Monomer | % m-LACT* | % L,L/D,D-LACT* |
| 1 | 155 | 0,5 | DCHA | 124 | & | 25 | 75 |
| 2 | 155 | 1 | DCHA | 124 | § | 20 | 80 |
| 3 | 155 | 1 | TMPIP | 98 | § | 20 | 80 |
| 4 | 155 | 1 | PMPIP | 108 | § | 19 | 81 |
| 5 | 155 | 1 | EDiPA | 90 | § | 20 | 80 |
| 6 | 155 | 1 | TBA | 129 | § | 24 | 76 |
| 7 | 155 | 1 | KBenz | 111 | § | 16 | 84 |
| 8 | 155 | 1 | MgO | 28 | § | 43 | 57 |
| 9 | 155 | 1 | CaO | 39 | § | 36 | 64 |

§ = keine Nebenprodukte
& = Nebenprodukte
KBenz = Kaliumbenzoat
* NMR-Daten; meso-Lactid: 90 % meso, 10 % L

**Tabelle 6**

| Racemisierung von L-Lactid | | | | | | | |
|---|---|---|---|---|---|---|---|
| Exp. Nr. | Temp. °C | Zeit h | Kat. | Mol Lac./Kat. | Monomer | % m-LACT* | % L, L/D, D-LACT* |
| 1 | 100 | 12 | DCHA | 20 | -- | 18 | 82 |
| 2 | 130 | 20 | TOA | 245 | -- | 24 | 76 |
| 3 | 130 | 1 | TOA | 10 | -- | 18 | 82 |
| 4 | 130 | 1 | TEA | 5 | 30 | 0#) | 100#) |
| 5 | 130 | 1 | TOA | 15 | 89 | 0#) | 100#) |

#) nach Waschen mit $H_2O$
TEA = Triethylamin
* NMR-Daten

**Tabelle 7**

| Racemisierung von Meso-Lactid | | | | | | | | |
|---|---|---|---|---|---|---|---|---|
| Exp. Nr. | Temp. °C | Zeit h | Kat. | Mol Lac./Kat. | Monomer* | % m-LACT@ | % L,L-LACT@ | % D, D-LACT@ |
| 1 | 140 | 2 | TBP | 694 | 98 | 79 | 13 | 8 |
| 2 | 140 | 6 | TBP | 718 | 94 | 42 | 30 | 28 |
| 3 | 140 | 24 | TBP | 690 | 89 | 31 | 35 | 34 |
| TBP = Tributylphosphin<br>* NMR-Daten; @ HPLC-Daten; Meso-Lactid: 96% Meso, 4% L | | | | | | | | |

**Beispiel 7 (Aufreinigung):**

Eine Aufreinigung der Reaktionsprodukte kann mittels fraktionierter Destillation und/oder Kristallisation erfolgen. Eine Kristallisation kann als Flüssig-Kristallisation oder als Schmelz-Kristallisation durchgeführt werden. Auf diese Weise ist nur eine Abtrennung des meso-Lactids möglich, L- und D-Lactid (Stereoisomere) können durch diese physikalischen Methoden nicht separiert werden. Der erfahrene Chemiker wird Ethylacetat oder Toluol für die Kristallisation von Lactid-Mischungen einsetzen. Auch Alkohole, Ketone etc. oder Mischungen hiervon können der Trennung dienen. Ein bevorzugtes Verfahren zur Abtrennung von Lactiden ist die Schmelz-Kristallisation.

In einer Kristallisationsvorrichtung wird die Schmelze des Lactide, die eine Temperatur von 135 °C aufweist, langsam abgekühlt und das verfestigte Material (Temperatur 125 °C) wird von den Wänden gesammelt, nachdem die Schmelze entfernt worden ist. Dieser Prozess wird mit dem gesammelten Material so lange wiederholt, bis die gewünschte Reinheit erreicht worden ist. Die Schmelztemperatur des racemischen Lactids beträgt 129 °C. Die restliche Schmelze kann in der Racemisierungsreaktion wieder verwendet werden und der Reinigungsprozess kann wiederholt werden.

## Patentansprüche

1. Verfahren zur Herstellung eines äquimolaren Gemisches aus den beiden Enantiomeren des Dilactids der Milchsäure, D,D-Dilactid und L,L-Dilactid, bei dem

    a) L-Milchsäure mit Trioctylamin zu Trioctylammoniumlactat umgesetzt wird,
    b) das Trioctylammoniumlactat in Gegenwart eines Katalysators destilliert wird, wobei eine Fraktion erhalten wird, die im wesentlichen aus den beiden Enantiomeren des Dilactids der Milchsäure, D,D-Dilactid und L,L-Dilactid, zusammengesetzt ist und noch D,L-Lactid (meso-Lactid) enthalten kann
    c) wobei die zuvor genannte Fraktion mit Aceton versetzt und damit umkristallisiert wird, wobei farblose Kristalle mit einem Schmelzpunkt von 112 bis 119 °C erhalten werden, die äquimolar aus D,D-Dilactid und L,L-Dilactid zusammengesetzt sind.

2. Verfahren nach Anspruch 1, **dadurch gekennzeichnet, dass** als Katalysator eine Organozinn-Verbindung verwendet wird.

3. Verfahren nach vorhergehendem Anspruch, **dadurch gekennzeichnet, dass** der Katalysator Dibutylzinnoxid ist, das in einer Dosierung von 0,1 bis 1 Massenprozent, bezogen auf das Gemisch im Destillationssumpf bei der destillativen Spaltung, eingesetzt wird.

4. Verfahren nach einem der vorhergehenden Ansprüche, **dadurch gekennzeichnet, dass** die L-Milchsäure mit Tri-n-octylamin zu Tri-n-octylammoniumlactat umgesetzt wird.

5. Verfahren nach einem der vorhergehenden Ansprüche, **dadurch gekennzeichnet, dass** die in Stufe b) erhaltene Fraktion zu jeweils 40 bis 50 Masseprozent aus D,D-Dilactid und L,L-Dilactid zusammengesetzt ist und ggf. als restlichen Bestandteil Mesolactid und/oder Oligolactide enthalten kann.

6. Verfahren nach einem der vorhergehenden Ansprüche, **dadurch gekennzeichnet, dass** die Destillation mittels einer Destillationskolonne durchgeführt wird.

7. Verfahren nach einem der vorhergehenden Ansprüche, **dadurch gekennzeichnet, dass** die Destillation im Vakuum durchgeführt wird.

## Claims

1. Method for manufacturing an equimolar mixture of the two enantiomers of the dilactide of lactic acid, D,D-dilactide and L,L-dilactide, in which

   a) L-lactic acid is converted with trioctylamine to form trioctyl ammonium lactate,
   b) the trioctyl ammonium lactate is distilled in the presence of a catalyst, a fraction being obtained which is composed essentially of the two enantiomers of the dilactide of lactic acid, D,D-dilactide and L,L-dilactide, and can also comprise D,L-lactide (meso-lactide),
   c) the previously mentioned fraction being mixed with acetone and hence recrystallised, colourless crystals with a melting point of 112 to 119°C being obtained, which are composed equimolarly of D,D-dilactide and L,L-dilactide.

2. Method according to claim 1, **characterised in that** an organotin compound is used as catalyst.

3. Method according to the preceding claim, **characterised in that** the catalyst is dibutyltin oxide which is used in a proportion of 0.1 to 1 per cent by mass relative to the mixture in the distillation sump during the distillative splitting.

4. Method according to one of the preceding claims, **characterised in that** the L-lactic acid is converted with tri-n-octylamine to form tri-n-octyl ammonium lactate.

5. Method according to one of the preceding claims, **characterised in that** the fraction obtained in step b) is composed of respectively up to 40 to 50 per cent by mass of D,D-dilactide and L,L-dilactide and possibly can comprise mesolactide and/or oligolactides as residual component.

6. Method according to one of the preceding claims, **characterised in that** the distillation is implemented by means of a distillation column.

7. Method according to one of the preceding claims, **characterised in that** the distillation is implemented in a vacuum.

## Revendications

1. Procédé de préparation d’un mélange équimolaire des deux énantiomères du dilactide de l’acide lactique, le D,D-dilactide et le L,L-dilactide, dans lequel

   a) l’acide L-lactique est mis en réaction avec de la trioctylamine pour donner du lactate de trioctylammonium,
   b) le lactate de trioctylammonium est distillé en présence d’un catalyseur, lors de quoi est obtenue une fraction qui est composée sensiblement des deux énantiomères du dilactide de l’acide lactique, le D,D-dilactide et le L,L-dilactide et qui peut encore contenir du D,L-lactide (méso-lactide),
   c) la fraction précitée étant additionnée d’acétone et ainsi recristallisée, lors de quoi on obtient, avec un point de fusion compris entre 112 et 119 °C, des cristaux incolores qui sont constitués de D,D-dilactide et de L,L-dilactide en proportion équimolaire.

2. Procédé selon la revendication 1, **caractérisé en ce que** comme catalyseur est employé un composé organostannique.

3. Procédé selon la revendication précédente, **caractérisé en ce que** le catalyseur est de l’oxyde de dibutyle-étain, qui est utilisé en un dosage de 0,1 à 1 % en masse, rapporté au mélange dans le bouilleur de distillation, lors de la dissociation par distillation.

4. Procédé selon l’une quelconque des revendications précédentes, **caractérisé en ce que** l’on fait réagir l’acide L-lactique avec de la tri-n-octylamine pour obtenir du lactate de tri-n-octylammonium.

5. Procédé selon l'une quelconque des revendications précédentes, **caractérisé en ce que** la fraction obtenue à l'étape b) est composée à raison de 40 à 50 % en masse respectivement de D,D-dilactide et de L,L-dilactide et peut contenir comme autres composants le méso-lactide et/ou des oligolactides.

6. Procédé selon l'une quelconque des revendications précédentes, **caractérisé en ce que** la distillation est effectuée au moyen d'une colonne de distillation.

7. Procédé selon l'une quelconque des revendications précédentes, **caractérisé en ce que** la distillation est effectuée dans le vide.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 198404311 A1
- EP 0657447 A


### In der Beschreibung aufgeführte Nicht-Patentliteratur


- **TINA M. QUITT ; GEOFFREY W. COATES.** *J. Am. Chem. Soc.,* 1999, vol. 121, 4072-4073
- *Macromolecules,* 2004, vol. 37 (18), 57-1862
- **SPASSKY.** *Macromol.Chem.Phys.,* 1996, vol. 197, 2672
- **OVITT ; COATES.** *J.Am.Chem.Soc.,* 2002, vol. 124, 1316
- **RADANO ; BAKER.** *J.Am.Chem.Soc.,* 2000, vol. 122, 1552